(19) Europäisches Patentamt
European Patent Office
Office européen des brevets

(11) Publication number: **0 411 884 A1**

# (12) EUROPEAN PATENT APPLICATION

(21) Application number: **90308381.4**

(22) Date of filing: **31.07.90**

(51) Int. Cl.5: **C07D 491/107**, G03C 1/685, G11B 7/24, //(C07D491/107, 311:00,209:00)

(30) Priority: **31.07.89 JP 198983/89**

(43) Date of publication of application: **06.02.91 Bulletin 91/06**

(84) Designated Contracting States: **DE FR GB**

(71) Applicant: **MATSUSHITA ELECTRIC INDUSTRIAL CO., LTD**
**1006, Ohaza Kadoma, Kadoma-shi**
**Osaka 571(JP)**

(72) Inventor: **Hibino, Junichi**
**Azuma Manshon 201, 46-2, Kuzuhanaka-machi**
**Hirakata-shi, Osaka(JP)**
Inventor: **Ando, Eiji**
**15-20 Fujigaoka 4-chome**
**Katano-shi, Osaka(JP)**

(74) Representative: **Myerscough, Philip Boyd et al**
**J.A. Kemp & Co. 14 South Square, Gray's InnInn**
**GB-London WC1R 5LX(GB)**

## (54) Novel photochromic spiropyran compounds.

(57) Novel photochromic spiropyran compounds having an absorption sensitivity in a longer wavelength region than known photochromic compounds are provided. The novel spiropyran compounds have a spiropyran skeleton having nitro groups at the 5′ and 6 positions, an alkyl group at the 1′ position and an alkanoyloxymethyl group at the 8 position.

EP 0 411 884 A1

# NOVEL PHOTOCHROMIC SPIROPYRAN COMPOUNDS

## BACKGROUND OF THE INVENTION

### Field of The Invention

This invention relates to photochromic materials and more particularly, to novel photochromic spiropyran compounds.

### Description of The Prior Art

Photochromic materials are known as those materials which undergo reversible changes in color. One of typical photochromic materials includes spiropyran compounds. A number of spiropyran compounds have been studied and developed up to now. When a spiropyran compound of the following formula (A) is irradiated with UV light with a wavelength of 340 nm, it is changed into the merocyanine of the formula (B) assuming a red color. When visible light with a wavelength of 580 nm is again applied to the merocyanine, it is returned to the spiropyran (A).

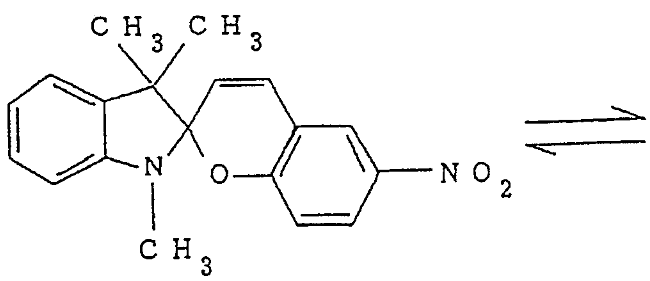

( A )

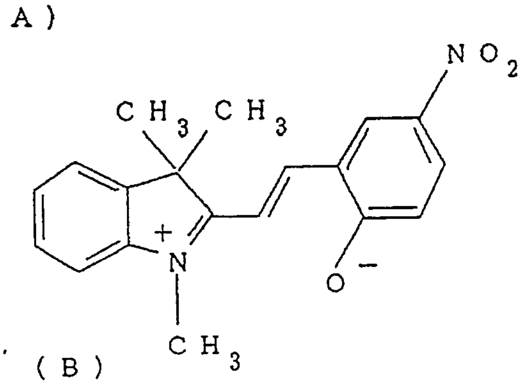

( B )

This property of the photochromic materials is suitable for application, for example, as optical recording media. In general, photochromic materials are thermally unstable either in an original state or in a converted state. For instance, the spiropyran compound (A) indicated above is lom in the stability when it is in the colored state. To overcome this drawback, it has been proposed, for example, in Japanese Laid-open Patent Application No. 59-224413 that an alkyl group is introduced into the spiropyran skeleton and an association product of the modified spiropyran is formed in the film formed by the Langmuir-Blodgett method, under which the resultant colored product has an improve stability. Although the colored product obtained from the spiropyran association product has high stability, it does not have high sensitivity in an oscillation region of semiconductor lasers. This makes it difficult to miniaturize devices.

## SUMMARY OF THE INVENTION

It is accordingly an object of the invention to provide novel photochromic spiropyran compounds which exhibit good absorption sensitivity to light in a longer wavelength range than known photochromic spiropyran compounds.

It is another object of the invention to provide novel spiropyran compounds which have a nitro group at the 5′ and 6 positions of the spiropyran skeleton, an alkyl group at the 1′ position and an alkanoyloxymethyl group at the 8 position.

According to the invention, there is provided a novel photochromic spiropyran compound of the following formula (I)

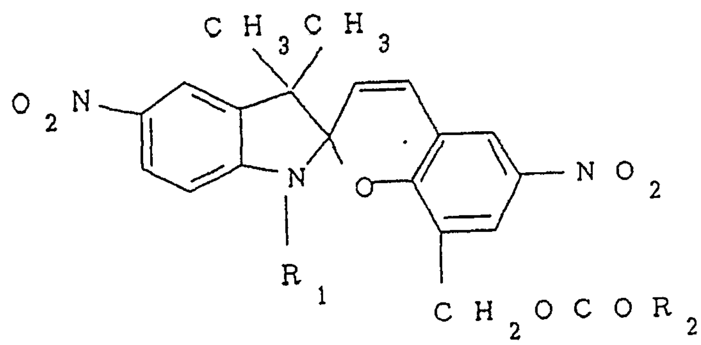

(I)

wherein $R_1$ represents an alkyl group having from 1 to 30 carbon atoms and $R_2$ represents an alkyl group having from 5 to 30 carbon atoms.

The spiropyran compound is advantageous over known spiropyran compounds in that the colored product obtained from the spiropyran compound of the invention is in an associated form which is thermally very stable and that the spiropyran compound has sensitivity in an oscillation range of semiconductor lasers, for example, of from 650 nm to 720 nm.

## BRIEF DESCRIPTION OF THE DRAWING

The sole figure is a UV to visible light absorption spectrum chart of a novel spiropyran compound according to the invention in a colorless state (A) and in a colored state (B) in polymethyl methacrylate (PMMA).

## DETAILED DESCRIPTION AND EMBODIMENTS OF THE INVENTION

Once again, the novel spiropyran compound of the invention has the following general formula

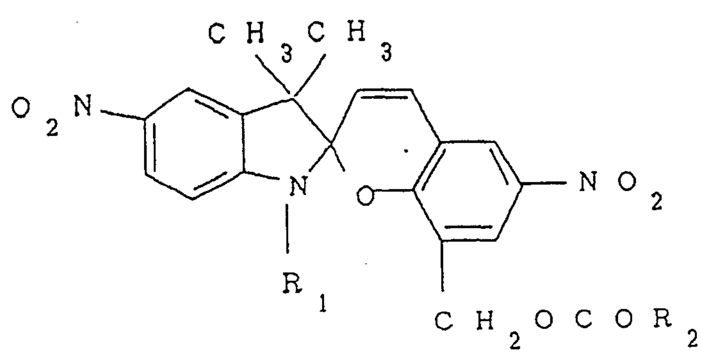

wherein $R_1$ represents an alkyl group having from 1 to 30 carbon atoms and $R_2$ represents an alkyl group having from 5 to 30 carbon atoms. In view of the sensitivity and the preparation in high yield, the alkyl group represented by $R_1$ has preferably from 6 to 30 carbon atoms, more preferably from 16 to 20 carbon atoms. Similarly, the alkyl group represented by $R_2$ has preferably from 16 to 24 carbon atoms. The preparation of the novel spiropyran compound of the invention is specifically described.

More particularly, the preparation of a spiropyran having the following structural formula (hereinafter abbreviated as NSP1822) according to a process including the following five steps is described step by step.

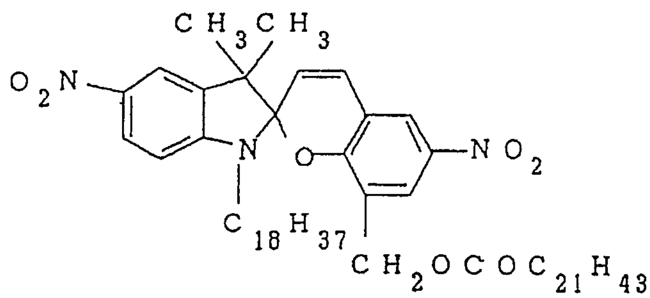

Step 1:

42.3 g (266 mmols) of 2,3,3-trimethyl indolenine of the formula ( 1 ) and 101.1 g (266 mmols) of iodooctadecane of the formula ( 2 ) are dissolved in 200 ml of 2-butanone, followed by heating under reflux for 40 hours. After removal of the 2-butanone by distillation, the resultant solid residue is recrystallized from 1000 ml of ethanol to obtain 91.5 g (197 mmols, yield 63.9%) of a reddish white solid of 1-octadecyl-2,3,3-trimethylindolenium iodide of the formula ( 3 ). The above reaction is shown below.

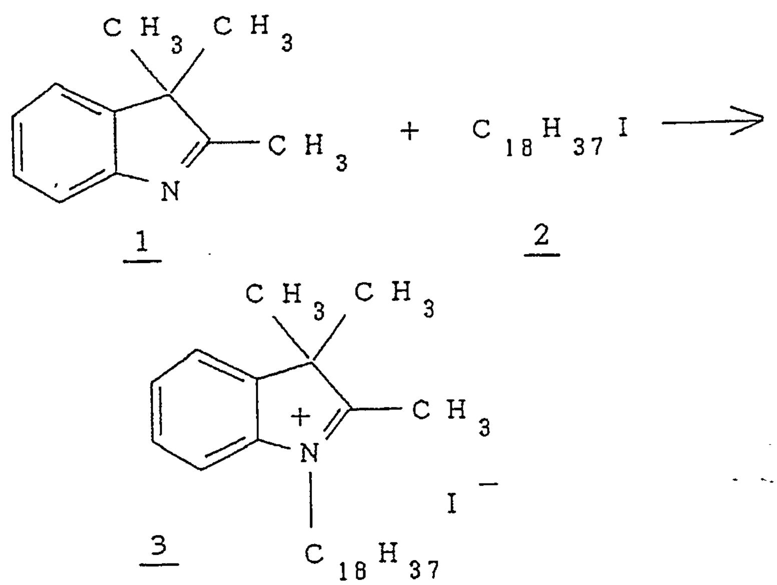

1

2

3

Step 2:

91.5 g (197 mmols) of a reddish white solid of 1-octadecyl-2,3,3-trimethylindolenium iodide of the formula ( 3 ) is dispersed in 100 ml of diethyl ether, followed by further dispersion in 400 ml of a 3.8N sodium hydroxide aqueous solution. After agitation for 3.5 hours, the resultant oil layer or phase is extracted

with diethyl ether, followed by drying over day and night with use of sodium hydroxide. Thereafter, the diethyl ether is distilled off to obtain 65.6 g (159 mmols, yield 80.7%) of a yellow liquid of 1-octadecyl-2-methylene-3,3-dimethylindoline of the formula ( 4 ) as shown below.

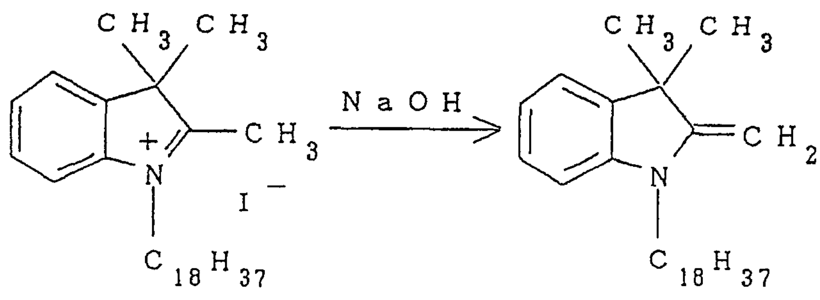


3 4

Step 3:

11.3 g (52.6 mmols) of 3-chloromethyl-5-methoxysalicylaldehyde of the formula ( 5 ) and 20.6 g (52.6 mmols) of silver behenate are mixed and heated under reflux in benzene (non-homogeneous system). After 40 hours, the reaction mixture is filtered and the resultant filtrate is concentrated, followed by recrystallization from 500 ml of a mixture of benzene and hexane at a mixing ratio by volume of 1:5 to obtain 11.1g (21.3 mmols, yield 40.5%) of yellow needle crystals of 3-docosanoylmethyl-5-nitrosalicylaldehyde of the formula ( 6 ) as shown below.

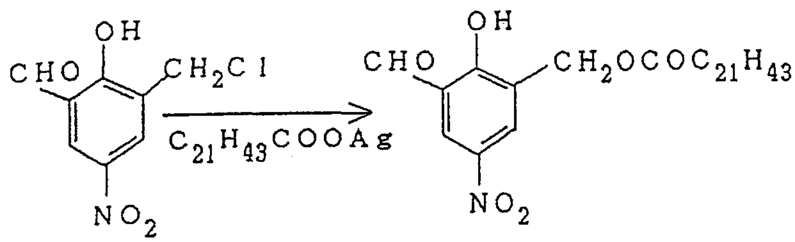


5 6

Step 4:

2 g (4.9 mmols) of the 1-octadecyl-2-methylene-3,3-dimethylindoline of the formula ( 4 ) obtained through Steps 1 and 2 and 2.1 g (4.1 mmols) of 3-docosanoylmethyl-5-nitrosalicylaldehyde obtained in Step 3 are heated under reflux in 20 ml of ethanol. The resultant dark green reaction solution is cooled and the resulting precipitate is recrystallized from 80 ml of ethanol three times to obtain 2.5 g (2.7 mmols, yield 65.9%) of spiropyran 7 as yellowish brown crystals. The reaction sequence is shown below.

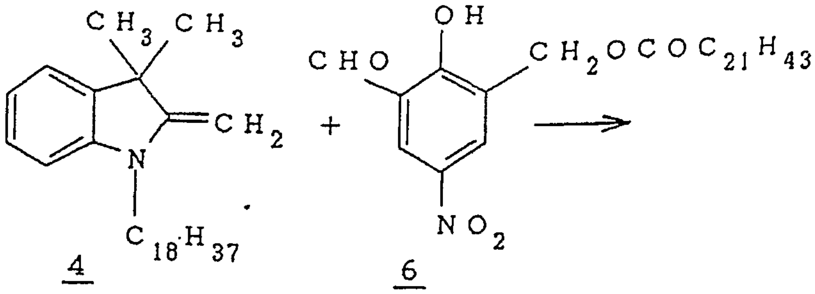

4 6

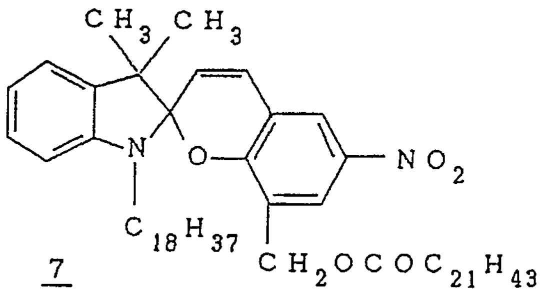

7

The thus obtained spiropyran is nitrated according to the procedure shown in the following step.

Step 5:

An acetic acid solution of 2.5 g (2.7 mmols) of spiropyran 7 is dropped into a mixture of 100 ml of acetic acid and 10 ml of fuming nitric acid. Fifteen minutes after the dropping, the mixture is poured into a mixture of hexane and water, followed by extraction with hexane. The organic layer is washed with sodium hydrogencarbonate and dried and concentrated, followed by purification with column chromatography and recrystallization twice with hexane to obtain 300 mg of nitrated spiropyran (NSP 1822). The above reaction is shown below.

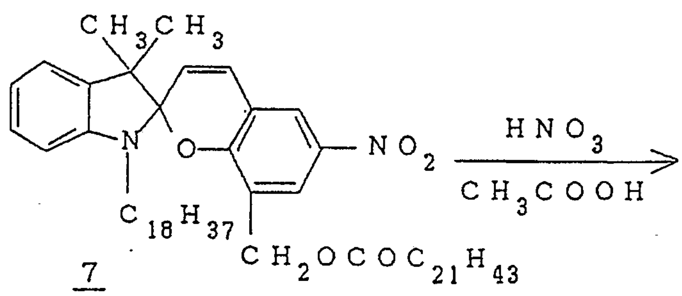


7

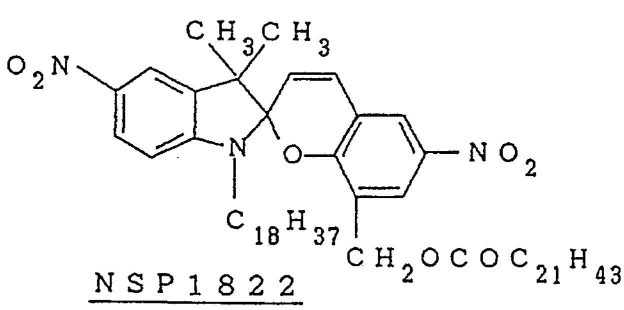

NSP1822

The chemical structure of the final product is confirmed by NMR. The attributions of the respective spectra of NMR are shown in Table 1 below.

Table 1

| Attributions of NMR of NSP1822 | | |
|---|---|---|
| Chemical Shift | Attribution | Number of H Atoms |
| 0.88(t) | methyl at an end of a long chain J = 6.8 Hz | 6 |
| 1.22(s) | 3′-methyl | 3 |
| 1.25(m) | long-chain methylene | 70 |
| 1.34(s) | 3′-methyl | 3 |
| 2.26(t) | methylene at the α position of the ester, J = 4.8 Hz | 2 |
| 3.14(m) | methylene at the α position of the indoline, J = 4.8 Hz | 2 |
| 4.91(s) | oxymethylene | 2 |
| 5.86(d) | 3-olefin | 1 |
| 6.53(d) | 7′-hydrogen | 1 |
| 6.98(d) | 4-olefin | 1 |
| 7.95(d) | 5-hydrogen, J = 2.0Hz | 1 |
| 8.01(d) | 7-hydrogen, J = 2.4Hz | 1 |
| 8.11(d) | 4′-hydrogen, J = 3.2Hz | 1 |
| 8.18(d,d) | 6′-hydrogen, J = 8.8, 2.4Hz | 1 |
| Note: The unit in the chemical shift is ppm and the letters in the parentheses indicate the shape of a peak , i.e. s: singlet, d: doublet, t: triplet, and m: multiplet, and the "J" in the attribution indicates a coupling constant. | | |

The spiropyran, NSP1822, and polymethyl methacrylate at a mixing ratio by weight of 1:1 are dissolved in methylene chloride and the resultant solution is spin coated on a quartz plate at 2000 rpm., thereby forming a thin film. when UV light (366 nm) is irradiated on the thin film, the colorless film is quickly turned into a colored film. The absorption maximum is 617 nm. From this, it will be seen that the maximum absorption is shifted to a longer side of wavelength as compared with recording media using known spiropyran compounds. The colored product is very stable: when it is allowed to stand at room temperature in the dark for 24 hours, no variation in the absorbance is observed. This material can be used for recording by the use of a semiconductor laser having a wavelength of 680 nm.

**In general compounds wherein $R_1$ is an alkyl group having from 1 to 30 carbon atoms may be obtained by the above procedure using iodo compounds from 1 to 30 carbon atoms in place of iodooctadecane in step 1.**

When the above procedure including Steps 1 to 5 described above is repeated except that iodopentane iodooctane and iodotriacontane are, respectively, used in the first step instead of the iodooctadecane, spiropran compounds of the formula (I) wherein $R_1$ represents $C_5H_{11}$, $C_6K_{17}$ and $C_{30}H_{61}$ are similarly obtained. With regard to spiropyran compounds of the formula (I) wherein $R_1$ is an alkyl group having not less than 31 carbon atoms, it is difficult to obtain such starting materials and thus, no experiments have not been conducted. However, we believe that similar results are probably obtained if such spiropyran compounds were prepared. Spiropyran compounds of the formula (I) wherein $R_1$ is any of $C_1$ to $C_4$ alkyl groups can be prepared in the same manner as described in Steps 1 to 5. Such spiropyran compounds are also stable for 10 hours or over when converted into colored products, respectively. The wavelength of absorption maximum of the respective colored products in polymethyl methacrylate are shown in Table 2.

**In general compounds wherein $R_2$ is an alkyl group having from 1 to 30 carbon atoms may be obtained by the above procedure using a silver carboxylate having from 1 to 30 carbon atoms in place of silver behenate in step 3.**

Moreover, when the above procedure including Steps 1 to 5 described above is also repeated except that silver acetate, silver hexoate and silver hentriacontaoate are used in Step 3 instead of silver behenate, spiropyran compounds of the formula (I) wherein $R_2$ represents $C_5H_{11}$ and $C_{30}H_{61}$ are similarly obtained. Similar to the case of $R_1$, we have not conducted any experiment using alkyl groups represented by $R_2$ and having not less than 30 carbon atoms since starting materials for such alkyl groups are difficult to obtain. Probably, similar results would be obtained if such spiropyran compounds were prepared.

The wavelength for absorption maximum for the respective colored products in polymethyl methacrylate are shown in Table as Nos.7 to 9.

These colored products are found to be very stable as in the products described before and no change in the absorbance is found when the colored products are allowed to stand at room temperature in the dark for 24 hours. These spiropyran materials can be used for recording by the use of a semiconductor laser beam with a wavelength of 670 nm.

Table 2

| | $R_1$ | $R_2$ | Absorption Maximum (nm) |
|---|---|---|---|
| 1 | $C_5H_{11}$ | $C_{21}H_{43}$ | 620 |
| 2 | $C_8H_{17}$ | $C_{21}H_{43}$ | 620 |
| 3 | $C_{18}H_{37}$ | $C_{21}H_{43}$ | 617 |
| 4 | $C_{30}H_{61}$ | $C_{21}H_{43}$ | 618 |
| 5 | $C_4H_9$ | $C_{21}H_{43}$ | 620 |
| 6 | $CH_3$ | $C_{21}H_{43}$ | 620 |
| 7 | $C_{18}H_{37}$ | $CH_3$ | 618 |
| 8 | $C_{18}H_{37}$ | $C_5H_{11}$ | 615 |
| 9 | $C_{18}H_{37}$ | $C_{30}H_{61}$ | 620 |

## Claims

1. A photochromic spiropyran compound of the following formula (I)

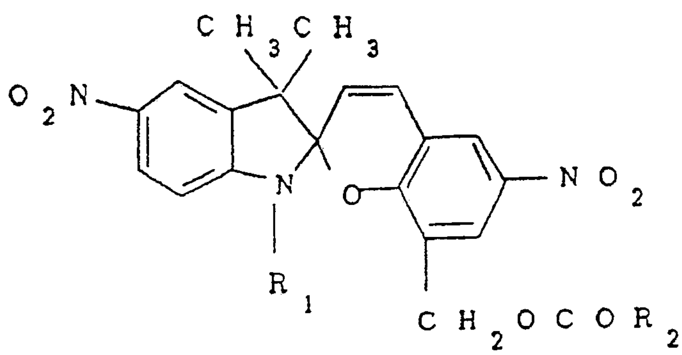

(I)

wherein $R_1$ represents an alkyl group having from 1 to 30 carbon atoms and $R_2$ represents an alkyl group having from 5 to 30 carbon atoms.

2. A photochromic spiropyran compound according to Claim 1, wherein $R_1$ represents an alkyl group having from 6 to 30 carbon atoms and $R_2$ represents an alkyl group having from 16 to 24 carbon atoms.

3. A process for the preparation of a compound of formula I, which comprises nitrating a compound of formula (II)

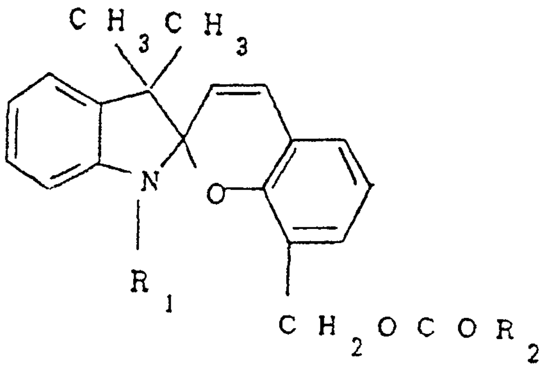

(II)

wherein $R_1$ and $R_2$ are as defined in claim 1.

4. An optical recording medium comprising a compound as claimed in claim 1.

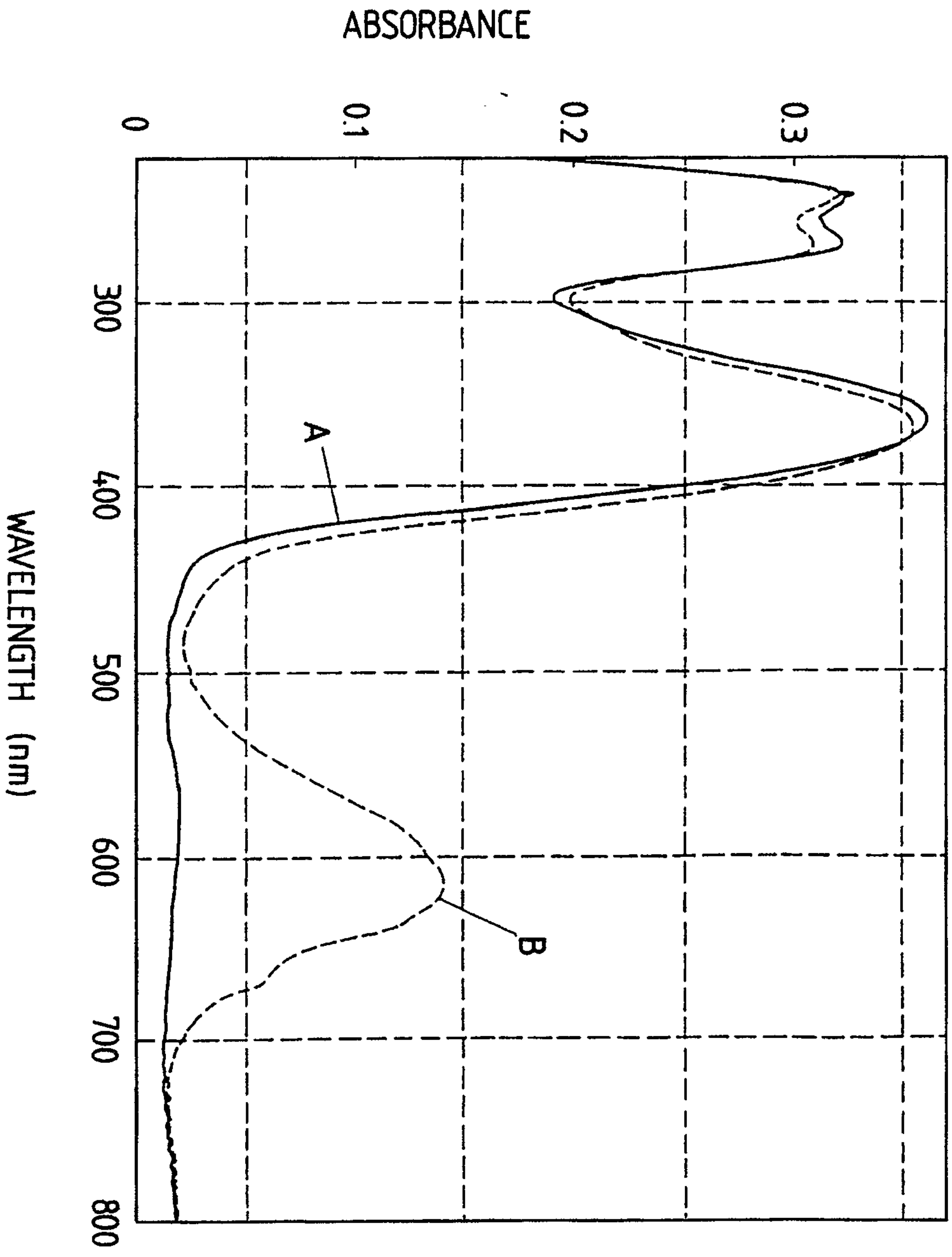
ABSORBANCE
0
0.1
0.2
0.3
300
400
500
600
700
800
WAVELENGTH (nm)
A
B

European Patent Office

# EUROPEAN SEARCH REPORT

Application Number

EP 90 30 8381

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y<br><br><br>D | EP-A-0179436 (MATSUSHITA ELECTRIC INDUSTRIAL CO., LTD.)<br>* claims 1, 3-6 *<br>& JP-A-61-116353<br>--- | 1, 2, 4 | C07D491/107<br>G03C1/685<br>G11B7/24//<br>(C07D491/107, 311:00,209:00) |
| Y | EP-A-0230024 (MATSUSHITA ELECTRIC INDUSTRIAL CO., LTD.)<br>* claims 1, 4 *<br>* example 1 *<br>--- | 1, 2, 4 | |
| Y | DE-A-1922842 (FUJI PHOTO FILM CO., LTD.)<br>* claims 1, 5 *<br>* page 7, example 4 *<br>--- | 1, 4 | |
| Y | DE-A-1769847 (AMERICAN CYANAMID CO.)<br>* claim 1 *<br>* example 5 *<br>----- | 1, 4 | |
| | | | TECHNICAL FIELDS SEARCHED (Int. Cl.5 ) |
| | | | C07D491/00<br>G03C1/00<br>G11B7/00 |

The present search report has been drawn up for all claims

| Place of search | Date of completion of the search | Examiner |
|---|---|---|
| BERLIN | 15 NOVEMBER 1990 | HASS C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)